# EUROPEAN PATENT SPECIFICATION

(11) **EP 4 410 339 B1**
(45) Date of publication and mention of the grant of the patent: **22.07.2026**
(21) Application number: 22907489.3
(22) Date of filing: 14.12.2022
(51) Int. Cl.: A61M 11/00, A61L 9/14, B05B 12/02, B05B 12/12, F24F 1/008, F24F 6/14

(54) **SPRAY DEVICE AND AIR TREATMENT DEVICE**
SPRÜHVORRICHTUNG UND LUFTBEHANDLUNGSVORRICHTUNG
DISPOSITIF DE PULVÉRISATION ET DISPOSITIF DE TRAITEMENT D'AIR

(30) Priority: 14.12.2021 JP 2021202619
(43) Date of publication of application: 07.08.2024
(73) Proprietor: Daikin Industries, Ltd., Osaka-shi, Osaka 530-0001 (JP)
(72) Inventor: ARAI, Junichiro, Osaka-shi, Osaka 530-0001 (JP); TAKEDA, Nobuaki, Osaka-shi, Osaka 530-0001 (JP); CHO, Joonsoo, Osaka-shi, Osaka 530-0001 (JP)
(74) Representative: Hoffmann Eitle
(86) International application number: PCT/JP2022/046074
(87) International publication number: WO 2023/112966

(56) References cited:
- WO-A1-2008/084864
- WO-A1-2008/084864
- WO-A1-2019/188791
- CN-A- 113 227 665
- JP-A- 2002 529 396
- JP-A- 2011 193 925
- JP-A- 2017 033 226
- JP-A- 2017 033 226
- JP-A- H1 085 315
- JP-U- 3 234 047
- US-A1- 2008 017 197
- US-B1- 6 263 872

## Description

### TECHNICAL FIELD

The present disclosure relates to a spray device and an air treatment device.

### BACKGROUND ART

As a means for people to easily obtain an antioxidative effect or an anti-inflammatory effect, a means for orally ingesting food (e.g., vegetables, fruits, supplements) containing components having an antioxidative effect and an anti-inflammatory effect has been known. JP 2020 110047 A discloses a method for producing broccoli processed food effectively containing sulforaphane as a component having an antioxidative effect.
US 6 263 872 B1 describes a portable air temperature controlling device useful for warming air surrounding an aerosolized drug formulation. Warming the air of an aerosol makes it possible to reduce the size of aerosol particles produced by an aerosol generation device. Additionally, warming the air forces the size of the aerosol particles to be in the range required for systemic drug delivery independent of ambient conditions. Smaller particles can be more precisely targeted to different areas of the respiratory tract.
US 2008/017197 A1 describes an inhaler with which the user inhales the medicine through a suction port which has a gas holding part in which exhaled air of the user or other air is to be stored, an air flow path which is connected with the gas holding part and guides the medicine to the suction port by inhalation of the user, and a medicine ejection part from which the medicine is to be ejected to the interior of the air flow path when the user inhales through the air flow path the exhaled air or other air stored in the gas holding part.
CN 113 227 665 A describes an environment control system which comprises an air blowing device that blows air toward a subject; and a control device that acquires at least one time from among the time when the subject plans to wake up, the time when the subject plans to sleep, the time of the sunrise, and the time of the sunset, and that switches the control of the air blowing device at the at least one time which was acquired.
JP 2011 193925 A describes a mist generator that includes a spray part to which water is supplied from the outside for squirting a mist to the space to be sprayed, a control part for controlling the spray part, a heat exchanger for heating the water supplied to the spray part, and a halt operation detecting part for detecting the halt of the heating operation of the outside heat source heating a fluid for the heat exchange which is supplied to the heat exchanger. The control part controls to restrict the spraying for halting the spraying of the spray part or for reducing the amount of spraying based on the detection of the halt of the heating operation of the outside heat source by the halt operation detecting part.
WO 2008/084864 A1 describes a capsaicin receptor activator comprising, as an active ingredient, a monoacylglycerol having a saturated or unsaturated fatty acid having to carbon atoms as a constituent fatty acid.
JP 2017 033226 A describes an aroma generation apparatus that is configured to, when a predetermined aroma generation time comes, determine a user situation based on biological information and/or peripheral environment information determined by a sensor; decide aroma information according to the determined user situation; and generate aroma based on the decided aroma information.

### SUMMARY OF THE INVENTION

### TECHNICAL PROBLEM

In the case where the antioxidative effect and the anti-inflammatory effect are obtained by orally ingesting food, useful substances having an antioxidative effect and an anti-inflammatory effect are absorbed by the body in the stage in which ingested food is digested in the body, thereby obtaining these effects. However, in such an oral ingestion, it takes time to digest the food, and the degree of digestion varies among individuals, and thus, the useful substances are not sufficiently absorbed into the body in some cases.

The present disclosure is intended to efficiently absorb useful substances having an antioxidative effect and an anti-inflammatory effect in the body.

### SOLUTION TO THE PROBLEM

The present invention is defined in independent claim 1. Distinct embodiments are defined in the dependent claims.

A first aspect is directed to a spray device including: a useful substance having an antioxidative effect or an anti-inflammatory effect; a sprayer (20) configured to spray the useful substance into a space (S) where a target person (T) is present; and a controller (30) configured to control the sprayer (20), wherein the useful substance sprayed by the sprayer (20) has a particle diameter from 1 µm to 10 µm inclusive. The spray device further includes: a humidity control unit (14) configured to control a humidity of air in the space (S); and a storage (31) configured to store data including the useful substance and a set humidity associated with the useful substance. The controller (30) acquires the set humidity associated with the useful substance sprayed by the sprayer (20) based on the data stored in the storage (31) and controls the humidity control unit (14) so as to satisfy the set humidity.

In the first aspect, the useful substance having a particle diameter from 1 µm to 10 µm inclusive is sprayed by the sprayer (20). Thus, the useful substance is captured on the nasal mucosa or oral mucosa of the target person (T). This allows the useful substance to be efficiently absorbed into the body through the nasal mucosa or oral mucosa. Furthermore, the controller (30) controls the humidity control unit (14) so as to satisfy the set humidity associated with the useful substance sprayed by the sprayer (20) based on the data stored in the storage (31). Accordingly, information on the humidity at which the useful substance efficiently acts on the target person (T) is stored in the storage (31). This allows the useful substance to be efficiently absorbed into the body of the target person (T).

A second aspect is an embodiment of the first aspect. In the second aspect, the useful substance sprayed by the sprayer (20) has a particle diameter from 5 µm to 10 µm inclusive.

In the second aspect, the useful substance having a particle diameter from 5 µm to 10 µm inclusive is sprayed by the sprayer (20). Thus, most of the useful substance is captured on the nasal mucosa without reaching the trachea or bronchi. This allows the useful substance to be efficiently absorbed into the body through the autonomic nervous system and the mucosal immune system of the person.

A third aspect is an embodiment of the first or second aspect. In the third aspect, the spray device further includes: a humidity control unit (14) configured to control a humidity of air in the space (S). The controller (30) controls the humidity control unit (14) such that the humidity of the space (S) becomes 50% or more during operation of the sprayer (20).

In the third aspect, the controller (30) controls the humidity control unit (14) to maintain the humidity of the space (S) at 50% or more during the operation of the sprayer (20). This makes it possible to prepare an environment in which the useful substance is easily absorbed into the body.

A fourth aspect of the present disclosure is an embodiment of the third aspect. In the fourth aspect, the controller (30) controls the humidity control unit (14) such that the humidity of the space (S) becomes 70% or more and 85% or less during the operation of the sprayer (20).

In the fourth aspect, the controller (30) controls the humidity control unit (14) to maintain the humidity of the space (S) at 70% or more and 85% or less during the operation of the sprayer (20). This makes it possible to prepare an environment in which the useful substance is more easily absorbed into the body.

A fifth aspect is an embodiment of any one of the first to fourth aspects. In the fifth aspect, the spray device further includes: a position sensor (16) configured to detect a position of nose of the target person (T); and a direction change unit (13) configured to change a direction in which the useful substance is sprayed by the sprayer (20). Tthe controller (30) drives the direction change unit (13) such that the useful substance is sprayed toward the nose of the target person (T) in accordance with a result of the detection of the position by the position sensor (16).

In the fifth aspect, the direction change unit (13) can change the direction in which the useful substance is sprayed, to the direction of the nose of the target person (T), so that the useful substance can reach the area around the nose of the target person (T). This allows the useful substance to be efficiently absorbed into the body.

A sixth aspect is an embodiment of any one of the first to fifth aspects. In the sixth aspect, the spray device further includes: a biosensor (15) configured to detect biological information of the target person (T). The useful substance has an antioxidative effect, and the controller (30) causes the sprayer (20) to spray the useful substance if it is determined based on the detected biological information acquired by the biosensor (15) that the parasympathetic nerve of the target person (T) is dominant.

In the sixth aspect, the useful substance having an antioxidative effect is sprayed when the parasympathetic nerve of the target person (T) is dominant. Thus, the target person (T) can efficiently obtain the antioxidative effect.

An seventh aspect of the present disclosure is an embodiment of the sixth aspect. In the seventh aspect, the useful substance includes at least one natural product selected from grape, black rice, blueberry, sesame, broccoli, garlic, spinach, tomato, watermelon, citrus, rosemary, oregano, thyme, sesame seed, tabasco pepper, pepper, ginger, plantago seed, and osbeckia chinensis.

A eighth aspect is an embodiment of the sixth or seventh aspect. **In** the eigth aspect, the useful substance includes at least one substance selected from anthocyanin, sesaminol, sulforaphane, allicin, lutein, lycopene, limonene, rosmanol, rosmari diphenol, protocatechuic acid, caffeic acid, rosmarinic acid, thymol, carvacrol, capsaicin, hydrocapsaicin, piperine, zingerone, gingerol, ginger all, geniposidic acid, and osbekic acid.

A ninth aspect is an embodiment of any one of the sixth to eighth aspects. **In** the ninth aspect, the useful substance includes sulforaphane, and the controller (30) causes the sprayer (20) to spray the useful substance so that a total spray amount of the sulforaphane in one day is from 10 µmol to 1000 µmol inclusive.

**In** the ninth aspect, the amount of the sulforaphane suitable for the target person to obtain a high antioxidative effect can be sprayed.

An tenth aspect is an embodiment of any one of the first to fifth aspects. **In** the tenth aspect, the spray device further includes: a biosensor (15) configured to detect biological information of the target person (T). The useful substance has an anti-inflammatory effect, and the controller (30) causes the sprayer (20) to spray the useful substance if it is determined based on the biological information detected by the biosensor (15) that the sympathetic nerve of the target person (T) is dominant.

**In** the tenth aspect, the useful substance having an anti-inflammatory effect is sprayed when the sympathetic nerve of the target person (T) is dominant. Thus, the target person (T) can efficiently obtain the anti-inflammatory effect.

A eleventh aspect is an embodiment of the tenth aspect. **In** the eleventh aspect, the useful substance includes at least one natural product selected from lemongrass, red thyme, patchouli, spearmint, eucalyptus, tea tree, true lavender, geranium bourbon, juniper, chamomile, German chamomile, frankincense, lemon, ginger, black pepper, clove, cinnamon, oregano, winter savory, star anise, lemon balm, citronella, melissa officinalis, lemon gum, and verbena.

A twelfth aspect of the present disclosure is an embodiment of the tenth or eleventh aspect. In the twelfth aspect, the useful substance includes at least one substance selected from citral, thymol, patchoulol, carvone, 1,8-cineole, terpinene-4-ol, linalool, geraniol, β-citronellol, α-pinene, chamazulene, farnesene, bisabolene, eugenol, carvacrol, anethole, citronellal, and neral.

A thirteenth aspect is an embodiment of any one of the tenth to twelfth aspects. In the thirteenth aspect, the useful substance includes a terpinene-4-ol, and the controller (30) causes the sprayer (20) to spray the useful substance so that a total spray amount of the terpinene-4-ol in one day is from 10 µmol to 1000 µmol inclusive.

In the thirteenth aspect, the amount of terpinene-4-ol suitable for the target person to obtain a high anti-inflammatory effect can be sprayed.

A fourteenth aspect is directed to an air treatment device including the spray device (10) of any one of the first to thirteenth aspects.

In the fourteenth aspect, the spray device (10) is applicable to the air treatment device (A).

### BRIEF DESCRIPTION OF THE DRAWINGS

FIG. 1 is a diagram schematically illustrating a general configuration of a spray device of a first embodiment.
FIG. 2 is a block diagram schematically illustrating the spray device.
FIG. 3 is a longitudinal sectional view of a sprayer.
FIG. 4 is a flowchart illustrating a process flow performed by a controller.
FIG. 5 is a flowchart of humidity control.
FIG. 6 is a diagram corresponding to FIG. 1 and illustrates a second embodiment.
FIG. 7 is a diagram corresponding to FIG. 4 and illustrates the second embodiment.
FIG. 8 corresponds to FIG. 1 and illustrates another embodiment.
FIG. 9 is a diagram corresponding to FIG. 2 and illustrates a third embodiment.
FIG. 10 is a schematic view of a control table of the third embodiment.
FIG. 11 is a diagram corresponding to FIG. 5 and illustrates the third embodiment.

### DESCRIPTION OF EMBODIMENTS

Embodiments of the present disclosure will be described in detail below with reference to the drawings. The present disclosure is not limited to the embodiments shown below, and various changes can be made within the scope without departing from the technical concept of the present disclosure. Since each of the drawings is intended to illustrate the present disclosure conceptually, dimensions, ratios, or numbers may be exaggerated or simplified as necessary for the sake of ease of understanding.

### <<First Embodiment>>

A spray device (10) of a first embodiment will be described.

### (1) Overview of Spray Device

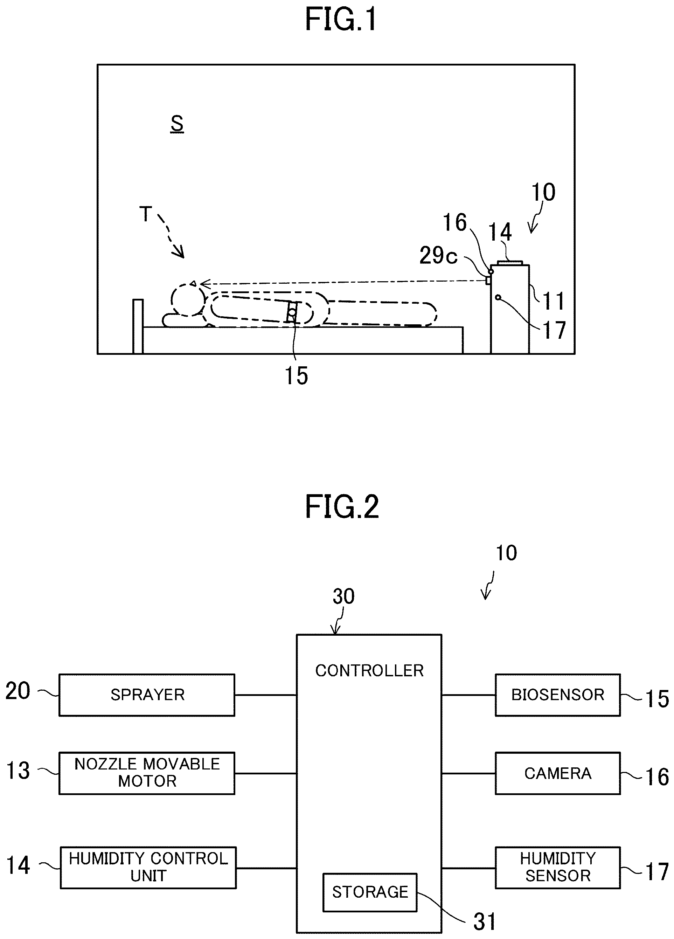

As illustrated in FIG. 1, the spray device (10) of this embodiment is placed in an indoor space (S) in which a target person (T) is present. The spray device (10) is installed on a floor surface of the indoor space (S). The indoor space (S) corresponds to a space of the present disclosure. The target person (T) sleeps on bedding such as a bed in the indoor space (S), for example.

The spray device (10) sprays a useful substance having an antioxidative effect or an anti-inflammatory effect toward the target person (T). The spray device (10) of this embodiment sprays a drug solution containing the useful substance having an antioxidative effect. Specifically, the useful substance contained in the drug solution contains sulforaphane as a substance having an antioxidative effect. Sulforaphane is a component mainly contained in broccoli sprouts and the like.

Part of oxygen taken in the human body by respiration or the like is changed to generate active oxygen. This active oxygen has a strong ability to oxidize cells, and if it is excessively generated in the body, it attacks normal cells. In contrast, humans have a function of suppressing the active oxygen (antioxidative function). The "antioxidative effect" in the present disclosure is not limited to an effect of suppressing oxidation of an object (for example, food or industrial raw materials) by the action of a substance itself, and includes an effect of enhancing an effect of reducing active oxygen (antioxidative function) originally possessed by humans by taking the substance into the body. Details of the useful substance having an antioxidative effect will be described later.

The drug solution of this embodiment may contain a substance other than the useful substance having an antioxidative effect. The useful substance merely needs to be blended in an amount sufficient to exhibit an antioxidative effect in the drug solution containing the useful substance, and the amount of each component blended in the drug solution is selected appropriately according, for example, to the other components to be blended together with the useful substance, and is not particularly limited.

The spray device (10) sprays the useful substance having a particle diameter from 1 µm to 10 µm inclusive toward the nose and the mouth of the target person (T). The "particle diameter" herein refers to a mean particle diameter. The spray device (10) sprays the useful substance having a predetermined particle diameter toward an area around the nose of the target person (T), so that the useful substance can be efficiently absorbed from the nasal mucosa of the target person (T). In the spray device (10) of this embodiment, the useful substance is sprayed toward the nose and mouth of the target person (T).

### (2) Details of Spray Device

As illustrated in FIGS. 1 to 3, the spray device (10) includes a casing (11), a sprayer (20), a nozzle movable motor (13), a humidity control unit (14), a biosensor (15), a camera (16), a humidity sensor (17), and a controller (30). In this embodiment, the sprayer (20), the nozzle movable motor (13), the humidity control unit (14), and the controller (30) are housed inside the casing (11).

### (2-1) Casing

The casing (11) is hollow. The casing (11) has a supply port (12). An atomized drug solution is supplied into an indoor space (S) through the supply port (12).

### (2-2) Sprayer

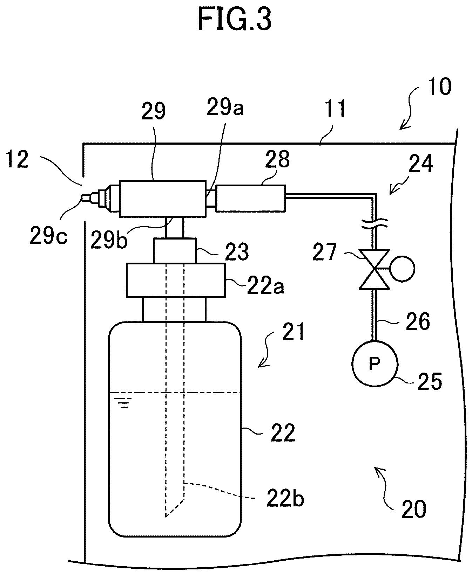

The sprayer (20) sprays the useful substance into the indoor space (S). As illustrated in FIG. 3, the sprayer (20) has a liquid reservoir (21), a gas supply section (24), and a two-fluid nozzle (29).

### (2-2-1) Liquid Reservoir

The liquid reservoir (21) supplies the drug solution stored therein to the two-fluid nozzle (29). The liquid reservoir (21) includes a tank (22) and a liquid-side joint (23). The tank (22) stores a drug solution. The tank (22) is connected to a two-fluid nozzle (29) via the liquid-side joint (23). A cap (22a) is attached to the top of the tank (22). A pipe (22b) is arranged inside the tank (22). The pipe (22b) sucks the drug solution in the tank (22) and sends the drug solution to the two-fluid nozzle (29). The tank (22) is detachable from the liquid-side joint (23).

One end of the liquid-side joint (23) is attached to the cap (22a) of the tank (22) and is connected to the upper end of the pipe (22b). The other end of the liquid-side joint (23) is connected to a liquid suction port (29b) of the two-fluid nozzle (29) to be described later. This allows the inside of the tank (22) and the two-fluid nozzle (29) to communicate with each other via the liquid-side joint (23).

### (2-2-2) Gas Supply Section

The gas supply section (24) supplies compressed air to the two-fluid nozzle (29). The gas supply section (24) includes an air pump (25), an air flow path (26), an electromagnetic valve (27), and a gas-side joint (28).

The air pump (25) sucks the air in the indoor space (S) and discharges the sucked air to the air flow path (26). The air pump (25) has a discharge portion connected to the air flow path (26).

The air flow path (26) connects the gas-side joint (28) and the air pump (25). The air flow path (26) of this embodiment is configured as a tube. The air which has passed through the air flow path (26) is supplied to the two-fluid nozzle (29) through the gas-side joint (28).

The electromagnetic valve (27) is arranged in the air flow path (26). The electromagnetic valve (27) is an on-off valve which opens and closes the air flow path (26). Instead of the electromagnetic valve (27), a flow rate control valve may be used which is capable of finely controlling the opening degree of the air flow path (26).

The gas-side joint (28) connects the air flow path (26) and the two-fluid nozzle (29). One end of the gas-side joint (28) is connected to an outflow end of air flow path (26). The other end of the gas-side joint (28) is connected to a gas inlet (29a) of the two-fluid nozzle (29) to be described later.

### (2-2-3) Two-Fluid Nozzle

The two-fluid nozzle (29) constitutes an atomization mechanism that atomizes the drug solution in the tank (22). Specifically, the two-fluid nozzle (29) atomizes the film of the drug solution using the shearing force of the airflow. The atomization mechanism may be of another type, such as a piezoelectric type, an electrostatic spraying type, or an ultrasonic type. The two-fluid nozzle (29) has a gas inlet (29a), a liquid suction port (29b), and a spray port (29c).

The gas inlet (29a) allows air to flow therein. The gas inlet (29a) is provided for the rear end of the two-fluid nozzle (29). The liquid suction port (29b) sucks the drug solution. The liquid suction port (29b) is provided for the lower portion of the two-fluid nozzle (29). The spray port (29c) discharges the atomized drug solution. The spray port (29c) is provided for the front end of the two-fluid nozzle (29). The spray port (29c) communicates with the supply port (12) of the casing (11).

The drug solution to be discharged through the spray port (29c) has a particle diameter from 1 µm to 10 µm inclusive. When the particle diameter of the drug solution is larger than 10 µm, the drug solution is not captured on the nasal mucosa or oral mucosa of the target person (T), and most of the drug solution passes through the throat and flows into the stomach. In this embodiment, the particle diameter of the drug solution is sufficiently small, so that the useful substance contained in the drug solution is easily captured on the nasal mucosa and oral mucosa of the target person (T). The useful substance captured on these mucosae enters the blood vessel or lymph and thus can act in the body.

The particle diameter of the drug solution discharged through the spray port (29c) is more preferably from 5 µm to 10 µm inclusive. The drug solution having a particle diameter from 5 µm to 10 µm inclusive is easily captured particularly on the nasal mucosa. When the particle diameter is less than 5 µm, the drug solution passes through the nasal cavity and then flows into the trachea and the bronchi to reach the pulmonary alveolus. In contrast, the drug solution having a particle diameter from 5 µm to 10 µm inclusive remains on and is absorbed in the nasal mucosa. The drug solution can thus act on the autonomic nervous system and the immune system in the body through the olfactory nerve from the nasal mucosa.

### (2-3) Nozzle Movable Motor

The nozzle movable motor (13) corresponds to a direction change unit of the present disclosure. The nozzle movable motor (13) shown in FIG. 2 changes the direction in which the useful substance is sprayed by the sprayer (20). The nozzle movable motor (13) is coupled with the two-fluid nozzle (29) via the shaft. When the shaft is rotated and driven by the nozzle movable motor (13), the direction of the two-fluid nozzle (29) is adjusted.

### (2-4) Humidity Control Unit

The humidity control unit (14) controls the humidity of the air in the indoor space (S). The humidity control unit (14) of this embodiment sucks the air in the indoor space (S) and humidifies the air. The humidity control unit (14) may be of any type, such as a vaporizing type, a heating type, a hybrid type, or an ultrasonic type, and is not particularly limited. The humidity control unit (14) may have a dehumidifying function of dehumidifying the air in the indoor space (S) in addition to the humidifying function.

### (2-5) Biosensor, Camera, and Humidity Sensor

The biosensor (15) detects signals that underlie the heart rate of the target person (T). The biosensor (15) may be of a contact type or a non-contact type. The biosensor (15) of this embodiment is a contact-type biosensor (15). Specifically, the biosensor (15) of this embodiment is a wearable sensor integral with a wristwatch, and is worn on the arm of the target person (T). The biosensor (15) may detect signals that underlie the biological information (e.g., a body temperature, blood pressure, blood sugar, body motion, or the like) other than the heart rate.

The camera (16) is attached to the casing (11), and captures an image of the target person (T) present in the indoor space (S). The camera (16) detects the positions of the nose and mouth of the target person (T). In other words, the camera (16) is a position sensor that identifies the positional information of predetermined portions of the target person (T). The camera (16) may detect only the position of the nose.

The camera (16) may detect information (e.g., biological information such as a respiratory cycle, a heart rate, a pulse wave, and a countenance) other than the positional information of the predetermined portions of the target person (T). In such a case, the camera (16) is a biosensor.

The humidity sensor (17) measures the humidity of the indoor space (S). The humidity sensor (17) of this embodiment is attached to the casing (11). The humidity sensor (17) may be separately installed in the indoor space (S).

### (2-6) Controller

The controller (30) includes a microcomputer mounted on a control board, and a memory device (specifically, a semiconductor memory) storing software for operating the microcomputer. As illustrated in FIG. 2, the controller (30) includes a storage (31). The controller (30) may include a storage (31) separate from the control board.

The storage (31) stores data including various types of useful substances and total spray amounts associated with the respective useful substances. The total spray amount associated with the useful substance refers to a total spray amount (cumulative spray amount) of the useful substance in one day (24 hours). The useful substance stored in the storage (31) may be a single substance, or may be a mixed substance in which multiple substances are mixed. The mixed substance may be different from other mixed substances in the mixing ratio of the substances contained.

As illustrated in FIG. 2, the controller (30) is connected to the sprayer (20), the nozzle movable motor (13), the humidity control unit (14), the biosensor (15), the camera (16), and the humidity sensor (17) in a wired or wireless manner, and is configured to be able to transmit and receive signals to and from these components.

The controller (30) controls a spray operation of the sprayer (20). Specifically, the controller (30) operates an air pump (25) and opens the electromagnetic valve (27) to spray the drug solution from the sprayer (20). The spray operation of the sprayer (20) may be performed continuously or intermittently.

The controller (30) controls the sprayer (20) based on the biological information (information of the heart rate) detected by the biosensor (15). In this embodiment, the controller (30) causes the sprayer (20) to spray the atomized drug solution if it is determined based on the biological information detected by the biosensor (15) that the parasympathetic nerve of the target person (T) is dominant. Accordingly, the useful substance is sprayed when the antioxidative effect is likely to be exhibited in the body, so that the target person (T) can obtain high antioxidative effect.

The controller (30) acquires, based on the data stored in the storage (31), information on the total spray amount in one day, associated with the useful substance stored in the sprayer (20), and controls the sprayer (20) such that the acquired, predetermined total spray amount of the useful substance is sprayed. In other words, the controller (30) stops the operation of the spray device (10) if the total spray amount of the useful substance sprayed from the sprayer (20) exceeds the predetermined total spray amount acquired from the storage (31) during a period from the predetermined starting time to the ending time which is after 24 hours from the predetermined starting time. The total spray amount of the useful substance may be calculated based on the cumulative time of the time during which the electromagnetic valve (27) is open from the predetermined start time to the ending time which is after 24 hours from the predetermined starting time, or may be calculated by other methods.

In this embodiment, the controller (30) sprays the drug solution from the sprayer (20) such that the total spray amount of sulforaphane in one day is from 10 µmol to 1000 µmol inclusive. This allows an appropriate amount of the useful substance for the target person (T) to obtain the effects of the useful substance to be sprayed.

The controller (30) drives the nozzle movable motor (13) such that the drug solution (useful substance) is sprayed toward the nose and mouth of the target person (T) in accordance with the results of detection of the positions of the nose and mouth by the camera (16). Thus, the useful substance can be efficiently absorbed through the nose and mouth of the target person (T).

The controller (30) controls the humidity control unit (14) such that the humidity of the indoor space (S) becomes 50% or more during operation of the sprayer (20). It is preferred that the controller (30) controls the humidity control unit (14) such that the humidity of the indoor space (S) is from 70% to 85% inclusive during operation of the sprayer (20). When the humidity of the indoor space (S) is 50% or more, the useful substance is easily absorbed into the body, and when the humidity of the indoor space (S) is from 70% to 85% inclusive, the useful substance is more easily absorbed into the body. Therefore, the controller (30) controls the humidity control unit (14) to prepare an environment in which the useful substance sprayed from the sprayer (20) is easily absorbed into the body.

The controller (30) may be provided in a place other than the casing (11). For example, the controller (30) may be provided in a server device connected to a local network, the Internet, and the like, or various communication terminals (a portable terminal, a personal computer, and the like).

### (3) Details of Useful Substance having Antioxidative Effect

The useful substance having an antioxidative effect, which is sprayed from the spray device (10), includes a natural product or a substance described below. In other words, as the useful substance having an antioxidative effect, whish is sprayed from the spray device (10), a substance other than sulforaphane may be employed. The useful substance having an antioxidative effect is not limited to a substance having an effect of reducing oxidization by the useful substance itself, and includes a substance having an effect of enhancing or accelerating an antioxidative function possessed by humans.

The useful substance of this embodiment includes at least one natural product selected from grape, black rice, blueberry, sesame, broccoli, garlic, spinach, tomato, watermelon, citrus, rosemary, oregano, thyme, sesame seed, tabasco pepper, pepper, ginger, plantago seed, and osbeckia chinensis.

The useful substance of this embodiment includes at least one substance selected from anthocyanins, sesaminol, sulforaphane, allicin, lutein, lycopene, limonene, rosmanol, rosmari diphenol, protocatechuic acid, caffeic acid, rosmarinic acid, thymol, carvacrol, capsaicin, hydrocapsaicin, piperine, zingerone, gingerol, ginger all, geniposidic acid, and osbekic acid.

The humans have a homeostasis function. The homeostasis function is a function in which a person tries to maintain his/her living body environment in a normal state, which is a function of maintaining a body environment in a comfortable, constant state at all times even when the person is infected with a virus or a bacterium which causes a disease, for example. The homeostasis function consists of the immune system, the autonomic nervous system, and the endocrine system, which are interacted with each other.

The activity of the sympathetic nerve, which is an autonomic nervous system, increases in a time zone in which the activity of the body increases in one day, and decreases in a time zone in which the activity of the body decreases in one day (circadian variation). **In** the time zone in which the activity of the sympathetic nerve increases, the lymphocytes are inhibited from escaping from the lymph node, and the number of lymphocytes in the lymph node increases. This increases the response of the immune system in the time zone in which the activity of the sympathetic nerve increases. On the other hand, in a time zone in which the activity of the sympathetic nerve is low (the parasympathetic nerve is dominant over the sympathetic nerve), lymphocytes escape from the lymph node and circulate in the body via lymph fluid and blood. This results in a decrease in the number of lymphocytes in the lymph node. This increases the response of the immune system in the time zone in which the activity of the sympathetic nerve increases.

Due to the nature of the homeostasis function of humans, when the activity of the sympathetic nerve increases (the sympathetic nerve is dominant), which increases the response of the immune system, the anti-inflammatory effect is efficiently exerted. On the other hand, when the activity of the sympathetic nerve is low (the parasympathetic nerve is dominant), which weakens the response of the immune system, the antioxidative effect is efficiently exerted. In this way, due to the nature of the homeostasis function of humans, in this embodiment, the useful substance having an antioxidative effect is sprayed toward the target person (T) when the parasympathetic nerve of the target person (T) is dominant.

### (4) Operation

The operation of the spray device will be described with reference to FIGS. 1 to 5.

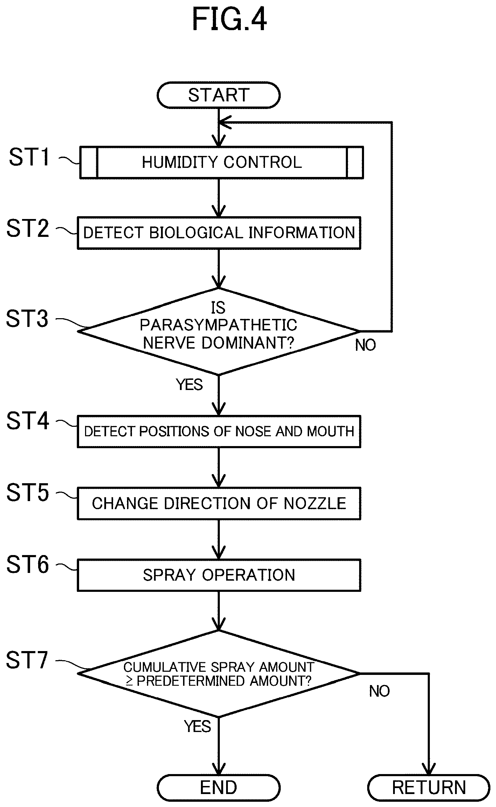

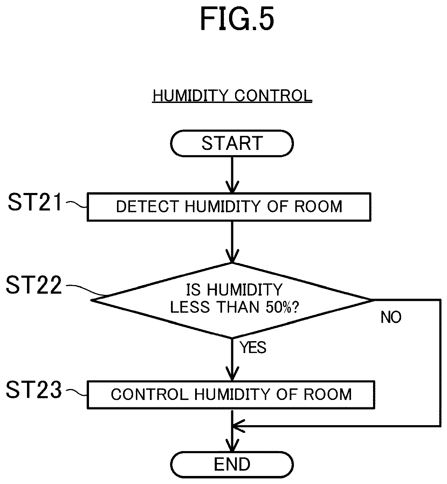

As illustrated in FIG. 4, when the spray device (10) is in an operating state, the controller (30) controls the humidity control unit (14) (Step ST1). Specifically, as illustrated in FIG. 5, the humidity sensor (17) detects a humidity of the indoor space (S) (Step ST21). Next, the controller (30) determines whether or not the humidity of the indoor space (S) detected by the humidity sensor (17) is less than 50% (Step ST22). When the humidity of the indoor space (S) is less than 50%, (YES in Step ST22), the controller (30) causes the humidity control unit (14) to humidify the indoor space (S) such that the humidity of the indoor space (S) becomes 50% or more. This makes it possible to prepare an environment in which the useful substance is easily absorbed into the body of the target person (T) during the operation of the sprayer (20).

As illustrated in FIG. 4, the biosensor (15) detects the heart rate of the target person (T) (Step ST2). Next, the controller (30) determines whether or not the parasympathetic nerve of the target person (T) is dominant (Step ST3). Specifically, the controller (30) acquires information on the RR interval of the heart rate detected by the biosensor (15) to determine whether or not the parasympathetic nerve of the target person (T) is dominant.

When the parasympathetic nerve of the target person (T) is dominant (YES in Step ST3), the camera (16) detects the positions of the nose and mouth of the target person (T) (Step ST4). Next, the controller (30) causes the direction of the two-fluid nozzle (29) to be changed. Specifically, the controller (30) controls the nozzle movable motor (13) based on the detected values by the camera (16) such that the spray port (29c) of the two-fluid nozzle (29) faces the nose and mouth of the target person (T). This allows the drug solution containing the useful substance to be sprayed toward the nose and mouth of the target person (T).

Next, the controller (30) causes the sprayer (20) to perform a spray operation of spraying the drug solution containing the useful substance (Step ST6). Specifically, in the spray operation, the air pump (25) is turned ON, the electromagnetic valve (27) is open, and the air transferred by the air pump (25) is introduced into the gas inlet (29a) of the two-fluid nozzle (29). At the same time, the drug solution stored in the tank (22) is sucked into the liquid suction port (29b) of the two-fluid nozzle (29) through the pipe (22b). In the two-fluid nozzle (29), the drug solution which has sucked through the liquid suction port (29b) is atomized by the airflow of air which has flowed in through the gas inlet (29a). The atomized drug solution is supplied to the outside of the casing (11) through the spray port (29c) and the supply port (12). The useful substance sprayed at this time by the sprayer (20) has a particle diameter from 5 µm to 10 µm inclusive.

According to this, the atomized useful substance is sprayed toward the nose and mouth of the target person (T), and thus is captured on the nasal mucosa and oral mucosa and is absorbed into the body. The useful substance is sprayed toward the nose and mouth of the target person (T), so that the amount of the useful substance used can be reduced compared to when it is emitted into the indoor space (S).

Then, the controller (30) determines whether or not the total spray amount (cumulative spray amount) of the useful substance in one day at this time is equal to or larger than the predetermined amount (Step ST7). In this embodiment, the controller determines whether or not the total spray amount of sulforaphane in one day at this time is 100 µmol or more. The sprayer (20) needs to perform a spray operation such that the total spray amount of sulforaphane in one day is from 10 µmol to 1000 µmol inclusive, and the upper limit of the total spray amount of sulforaphane in one day shown herein is a mere example.

When the cumulative spray amount of the useful substance sprayed from the sprayer (20) is a predetermined amount or more (YES in Step ST7), the controller (30) stops the operation of the spray device (10). When the cumulative spray amount of the useful substance sprayed from the sprayer (20) is the predetermined amount or more (NO in Step ST7), the controller (30) returns to Step ST1. This allows the useful substance to be kept from being sprayed more than necessary.

### (5) Features

(5-1)
The useful substance sprayed by the sprayer (20) has a particle diameter from 1 µm to 10 µm inclusive. According to this, the useful substance to be sprayed is atomized to have a predetermined particle diameter, and thus, the useful substance is easily captured on the nasal mucosa or oral mucosa of the target person (T). This allows the useful substance to be efficiently absorbed into the body.

(5-2)
The useful substance sprayed by the sprayer (20) has a particle diameter from 5 µm to 10 µm inclusive. When the particle diameter of the useful substance is less than 5 µm, most of the useful substance flows into the trachea or bronchi through the nasal cavity or oral cavity of the target person (T). In this embodiment, the useful substance has a particle diameter from 5 µm to 10 µm inclusive. Thus, the useful substance is captured on the nasal mucosa and oral mucosa without reaching the trachea or bronchi. In particular, the useful substance having a particle diameter from 5 µm to 10 µm inclusive is more likely to be captured on the nasal mucosa. As a result, the useful substance can be efficiently absorbed into the body through the autonomic nervous system and mucosal immune system.

(5-3)
The controller (30) controls the humidity control unit (14) such that the humidity of the indoor space (S) becomes 50% or more during operation of the sprayer (20). According to this, the humidity of the indoor space (S) is maintained at 50% or more during the operation of the sprayer (20). This makes it possible to prepare an environment in which the useful substance is easily absorbed into the body.

(5-4)
The controller (30) controls the humidity control unit (14) such that the humidity of the indoor space (S) is from 70% to 85% inclusive during operation of the sprayer (20). This makes it possible to maintain the indoor space (S) at a humidity from 70% to 85% inclusive during operation of the sprayer (20), and thus, an environment in which the useful substance is easily absorbed into the body can be prepared.

(5-5)
The controller (30) drives the nozzle movable motor (13) such that the useful substance is sprayed toward the nose and mouth of the target person in accordance with the results of detection of the positions by the camera (16). This allows the useful substance to reach an area around the nose and mouth of the target person (T), and hence allows the useful substance to be absorbed efficiently into the body through the nasal mucosa and oral mucosa.

In addition, the useful substance is sprayed over an area around the nose and mouth of the target person (T). Thus, the amount of the useful substance used can be reduced as compared with the case where the useful substance is sprayed into the indoor space (S). As a result, coloring due to adhesion of the useful substance to walls and clothes can be reduced, and the cost of the useful substance can be reduced.

(5-6)
When it is determined that the parasympathetic nerve of the target person (T) is dominant based on the biological information detected by the biosensor (15), the controller (30) causes the sprayer (20) to spray the useful substance having an antioxidative effect. According to this, the useful substance having an antioxidative effect is sprayed when the parasympathetic nerve of the target person (T) is dominant. Thus, the target person (T) can efficiently obtain the antioxidative effect.

(5-7)
The controller (30) causes the sprayer (20) to spray the useful substance such that a total spray amount of the sulforaphane in one day is from 10 µmol to 1000 µmol inclusive. This allows an appropriate amount of sulforaphane for the target person (T) to obtain the antioxidative effect to be sprayed.

### <<Second Embodiment>>

A spray device (10) of a second embodiment will be described. The spray device (10) of this embodiment is the spray device (10) of the first embodiment wherein the useful substance contained in the drug solution is changed to a useful substance having an anti-inflammatory effect. Thus, the following description will be focused on the differences of the spray device (10) of this embodiment from the spray device (10) of the first embodiment.

### (1) Spray Device

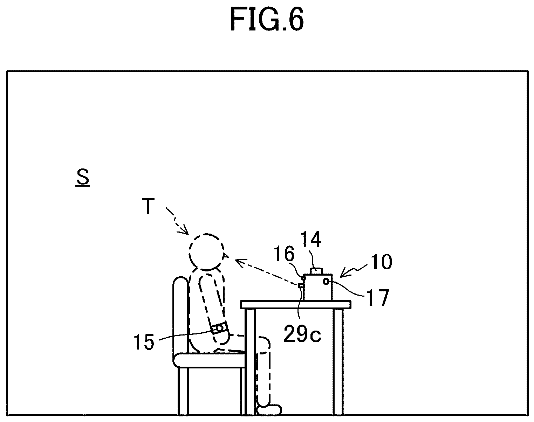

As illustrated in FIG. 6, in this embodiment, the target person (T) sits on a chair in the indoor space (S), for example. The spray device (10) is disposed on a desk placed in front of the target person (T).

A drug solution of this embodiment contains a useful substance having an antioxidative effect. Specifically, the drug solution contains terpinene-4-ol as the substance having an anti-inflammatory effect. Terpinene-4-ol is obtained as an extract from leaves, branches, and bark of tea tree.

In this embodiment, the controller (30) causes the sprayer (20) to spray the atomized drug solution if it is determined based on the biological information (information about the heart rate) detected by the biosensor (15) that the sympathetic nerve of the target person (T) is dominant. Accordingly, the useful substance is sprayed when the anti-inflammatory effect is easily exerted in the body, so that the target person (T) can obtain high anti-inflammatory effect.

The controller (30) causes the sprayer (20) to spray the drug solution such that a total spray amount of the terpinene-4-ol in one day is from 10 µmol to 1000 µmol inclusive, based on the data stored in the storage (31).

### (2) Details of Useful Substance having Anti-Inflammatory Effect

The useful substance having an anti-inflammatory effect, which is sprayed from the spray device (10), includes a natural product or a substance described below. **In** other words, as the useful substance having an anti-inflammatory effect, which is sprayed from the spray device (10), a substance other than terpinene-4-ol may be employed.

The useful substance of this embodiment includes at least one natural product selected from lemongrass, red thyme, patchouli, spearmint, eucalyptus, tea tree, true lavender, geranium bourbon, juniper, chamomile, German chamomile, frankincense, lemon, ginger, black pepper, clove, cinnamon, oregano, winter savory, star anise, lemon balm, citronella, melissa officinalis, lemon gum, and verbena.

The useful substance of this embodiment includes at least one substance selected from citral, thymol, patchoulol, carvone, 1,8-cineole, terpinene-4-ol, linalool, geraniol, beta-citronellol, alpha-pinene, chamazulene, farnesene, bisabolene, eugenol, carvacrol, anethole, citronellal, and neral.

### (3) Operation

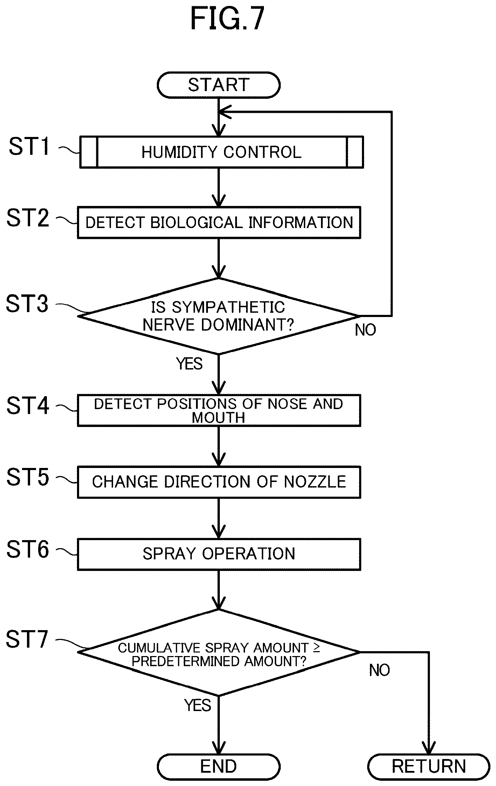

As illustrated in FIG. 7, the operation of the spray device (10) of this embodiment is different from the operation of the spray device (10) of the first embodiment in Steps ST3 and ST7.

In Step ST3, the controller (30) determines whether or not the sympathetic nerve of the target person (T) is dominant. Specifically, the controller (30) acquires information on the RR interval of the heart rate detected by the biosensor (15) to determine whether or not the sympathetic nerve of the target person (T) is dominant. When the sympathetic nerve of the target person (T) is dominant (YES in Step ST3), the camera (16) detects the positions of the nose and mouth of the target person (T) (Step ST4).

In Step ST7, the controller (30) determines whether or not the total spray amount (cumulative spray amount) of the useful substance in one day at this time is equal to or larger than the predetermined amount. In this embodiment, the controller determines whether or not the total spray amount of terpinene-4-ol in one day at this time is 100 µmol or more. The sprayer (20) needs to perform a spray operation such that the total spray amount of terpinene-4-ol in one day is from 10 µmol to 1000 µmol inclusive, and the upper limit of the total spray amount of sulforaphane in one day shown herein is a mere example.

### (4) Features

(4-1)
If it is determined that the sympathetic nerve of the target person (T) is dominant based on the biological information detected by the biosensor (15), the controller (30) causes the sprayer (20) to spray the useful substance having an anti-inflammatory effect. According to this, the useful substance having an anti-inflammatory effect is sprayed when the sympathetic nerve of the target person (T) is dominant. Thus, the target person (T) can efficiently obtain the anti-inflammatory effect.

(4-2)
The controller (30) causes the sprayer (20) to spray the useful substance such that a total spray amount of the terpinene-4-ol in one day is from 10 µmol to 1000 µmol inclusive. This allows an appropriate amount of terpinene-4-ol for the target person (T) to obtain the anti-inflammatory effect to be sprayed.

### <<Third Embodiment>>

A spray device (10) of a third embodiment will be described. The spray device (10) of this embodiment is the spray device (10) of the first embodiment in which the controller (30) has been changed. Thus, the following description will be focused on the differences of the spray device (10) of this embodiment from the spray device (10) of the first embodiment.

### (1) Controller

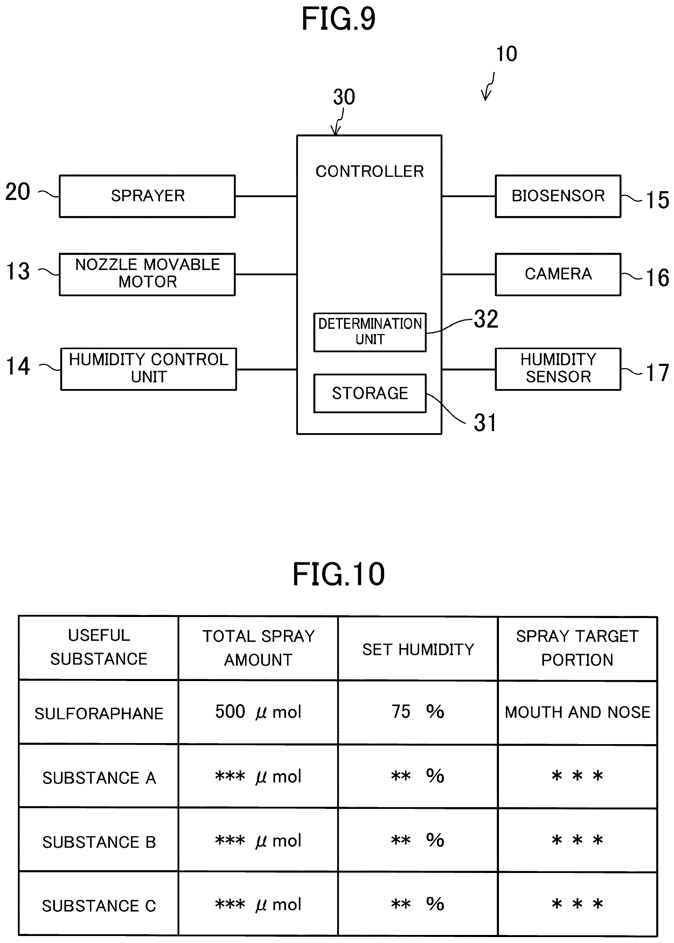

As illustrated in FIG. 9, the controller (30) includes a storage (31) and a determination unit (32).

The storage (31) stores a control table. As illustrated in FIG. 10, the control table defines data including a plurality of kinds of useful substances, the total spray amounts and the set humidities associated with the respective useful substances. The total spray amount used here is a total spray amount (cumulative spray amount) of the useful substance in one day (24 hours) as in the first embodiment. The set humidity is a humidity at which the useful substance efficiently acts on the target person (T). The total spray amount and the set humidity vary for each associated one of the useful substances. The set humidity is defined to be 50% or more. The set humidity is defined to be preferably from 70% to 85% inclusive. Numerical values shown in FIG. 10 are mere examples, and not limited thereto.

The control table may define, in addition to the total spray amounts and the set humidities associated with the respective useful substances, set temperatures associated with the respective useful substances, the concentrations of the useful substances, spray target portion, and the like. The spray target portion is a part of the body set to allow the useful substance to efficiently act on the target person (T). For example, when the mouth and nose are set as spray target portions, the useful substance is sprayed toward the mouth and nose of the target person (T). In other words, the data of the spray target portion is information indicating the spray direction.

The determination unit (32) determines the useful substance to be sprayed from the sprayer (20). For example, when the target person (T) inputs a specific useful substance by means of a switch or touch panel button, or when the tank (22) of the spray device (10) is attached to the liquid-side joint (23), the determination unit (32) acquires information on the type of the useful substance to be sprayed and determines the useful substance.

When the useful substance to be sprayed is determined, the controller (30) acquires information on the total spray amount and the set humidity associated with the useful substance based on the useful substance determined and the control table of FIG. 10. The controller (30) controls the sprayer (20) such that the acquired total spray amount of the useful substance is sprayed. The controller (30) controls the humidity control unit (14) so as to satisfy the acquired set humidity.

### (2) Operation

The operation of the spray device (10) of this embodiment differs from the operation of the spray device (10) of the first embodiment in humidity control. The humidity control of this embodiment will be described below.

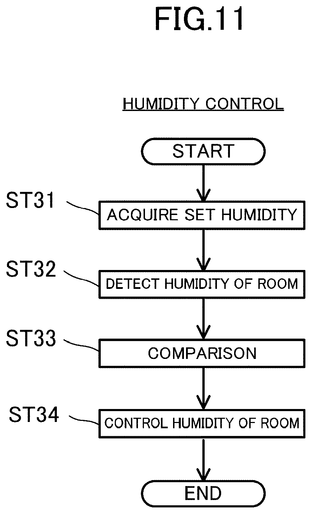

As illustrated in FIG. 11, the controller (30) acquires a set humidity associated with the determined useful substance based on the data stored in the storage (31) (Step ST31). Next, the controller (30) detects a humidity of the indoor space (S) based on an output from the humidity sensor (17) (Step ST32).

Then, the controller compares the acquired set humidity with the humidity of the indoor space (S) (Step ST33). Next, based on the result of the comparison in step ST33, the controller (30) controls the humidity control unit (14) such that the humidity of the indoor space (S) is the set humidity (Step ST34). As described above, the humidity of the indoor space (S) is controlled to the humidity at which the target person (T) easily absorbs the predetermined useful substance (U), so that the useful substance can be efficiently absorbed into the body of the target person (T).

### (3) Variations

Although the differences of the embodiments from the first embodiment have been described above, the useful substance contained in the drug solution for the spray devices (10) of each of the embodiments may be changed to the useful substance having an anti-inflammatory effect shown in the second embodiment.

### «Other Embodiments»

The above embodiments may be modified as follows. In the following description, differences from each embodiment will be described.

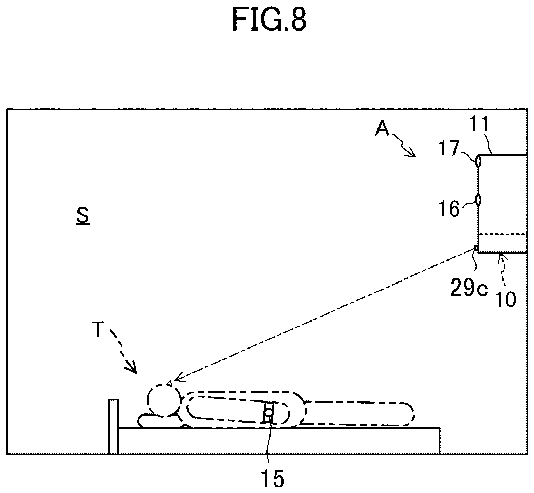

The spray device (10) of each of the embodiments may be installed in an air treatment device. Specifically, for example, as illustrated in FIG. 8, the spray device (10) may be integral with the air conditioner as the air treatment device (A). The air conditioner controls the temperature of air in the indoor space (S). In such a case, the controller (30) may control the air treatment device (A) such that the temperature of the indoor space (S) becomes a temperature at which the useful substance is easily absorbed into the body during the operation of the sprayer (20). The air treatment device (A) and the spray device (10) may be provided separately from each other.

The position sensor (16) of the spray device (10) of each embodiment may not be attached to the casing (11), and may be attached to the ceiling or lighting device.

The spray device (10) of each embodiment sprays a drug solution containing a useful substance, but may spray a powdered solid containing the useful substance.

The spray device (10) of each embodiment may include a plurality of tanks (22). In such a case, the respective tanks (22) may store a drug solution containing a useful substance having an antioxidative effect and a drug solution containing a useful substance having an anti-inflammatory effect. The respective tanks (22) may store drug solutions containing useful substances having different kinds of antioxidative effects or drug solutions containing useful substances having different kinds of anti-inflammatory effect.

While the embodiments and variations thereof have been described above, it will be understood that various changes in form and details may be made without departing from the scope of the claims. The elements according to embodiments, the variations thereof, and the other embodiments may be combined and replaced with each other.

### INDUSTRIAL APPLICABILITY

As can be seen from the foregoing description, the present disclosure is useful for a spray device and an air treatment device.

### DESCRIPTION OF REFERENCE CHARACTERS

- 10: Spray Device
- 13: Nozzle Movable Motor (Direction Change Unit)
- 14: Humidity Control Unit
- 15: Biosensor
- 16: Camera (Position Sensor)
- 20: Sprayer
- 30: Controller
- 31: Storage
- A: Air Treatment Device
- S: Indoor Space (Space)
- T: Target Person

## Claims

1. A spray device comprising:
a useful substance having an antioxidative effect or an anti-inflammatory effect;
a sprayer (20) configured to spray the useful substance into a space (S) where a target person (T) is present;
a controller (30) configured to control the sprayer (20);
a humidity control unit (14) configured to control a humidity of air in the space (S); and
a storage (31) configured to store data including the useful substance and a set humidity associated with the useful substance, wherein
the useful substance sprayed by the sprayer (20) has a particle diameter from 1 µm to 10 µm inclusive, and
the controller (30) acquires the set humidity associated with the useful substance sprayed by the sprayer (20) based on the data stored in the storage (31) and controls the humidity control unit (14) so as to satisfy the set humidity.

2. The spray device of claim 1, wherein
the useful substance sprayed by the sprayer (20) has a particle diameter from 5 µm to 10 µm inclusive.

3. The spray device of claim 1 or 2, further comprising:
a humidity control unit (14) configured to control a humidity of air in the space (S), wherein
the controller (30) controls the humidity control unit (14) such that the humidity of the space (S) becomes 50% or more during operation of the sprayer (20).

4. The spray device of claim 3, wherein
the controller (30) controls the humidity control unit (14) such that the humidity of the space (S) becomes 70% or more and 85% or less during the operation of the sprayer (20).

5. The spray device of any one of claims 1 to 4, further comprising:
a position sensor (16) configured to detect a position of nose of the target person (T); and
a direction change unit (13) configured to change a direction in which the useful substance is sprayed by the sprayer (20), wherein
the controller (30) drives the direction change unit (13) such that the useful substance is sprayed toward the nose of the target person (T) in accordance with a result of the detection of the position by the position sensor (16).

6. The spray device of any one of claims 1 to 5, further comprising:
a biosensor (15) configured to detect biological information of the target person (T), wherein
the useful substance has an antioxidative effect, and
the controller (30) causes the sprayer (20) to spray the useful substance if it is determined based on the biological information detected by the biosensor (15) that the parasympathetic nerve of the target person (T) is dominant.

7. The spray device of claim 6, wherein
the useful substance includes at least one natural product selected from grape, black rice, blueberry, sesame, broccoli, garlic, spinach, tomato, watermelon, citrus, rosemary, oregano, thyme, sesame seed, tabasco pepper, pepper, ginger, plantago seed, and osbeckia chinensis.

8. The spray device of claim 6 or 7, wherein
the useful substance includes at least one substance selected from anthocyanin, sesaminol, sulforaphane, allicin, lutein, lycopene, limonene, rosmanol, rosmari diphenol, protocatechuic acid, caffeic acid, rosmarinic acid, thymol, carvacrol, capsaicin, hydrocapsaicin, piperine, zingerone, gingerol, ginger all, geniposidic acid, and osbekic acid.

9. The spray device of any one of claims 6 to 8, wherein
the useful substance includes sulforaphane, and
the controller (30) causes the sprayer (20) to spray the useful substance so that a total spray amount of the sulforaphane in one day is from 10 µmol to 1000 µmol inclusive.

10. The spray device of any one of claims 1 to 5, further comprising:
a biosensor (15) configured to detect biological information of the target person (T), wherein
the useful substance has an anti-inflammatory effect, and
the controller (30) causes the sprayer (20) to spray the useful substance if it is determined based on the biological information detected by the biosensor (15) that the sympathetic nerve of the target person (T) is dominant.

11. The spray device of claim 10, wherein
the useful substance includes at least one natural product selected from lemongrass, red thyme, patchouli, spearmint, eucalyptus, tea tree, true lavender, geranium bourbon, juniper, chamomile, German chamomile, frankincense, lemon, ginger, black pepper, clove, cinnamon, oregano, winter savory, star anise, lemon balm, citronella, melissa officinalis, lemon gum, and verbena.

12. The spray device of claim 10 or 11, wherein
the useful substance includes at least one substance selected from citral, thymol, patchoulol, carvone, 1,8-cineole, terpinene-4-ol, linalool, geraniol, β-citronellol, α-pinene, chamazulene, farnesene, bisabolene, eugenol, carvacrol, anethole, citronellal, and neral.

13. The spray device of any one of claims 10 to 12, wherein
the useful substance includes a terpinene-4-ol, and
the controller (30) causes the sprayer (20) to spray the useful substance so that a total spray amount of the terpinene-4-ol in one day is from 10 µmol to 1000 µmol inclusive.

14. An air treatment device comprising the spray device (10) of any one of claims 1 to 13.

## Patentansprüche

1. Sprühvorrichtung, umfassend:
eine Nutzsubstanz mit einer antioxidativen Wirkung oder einer entzündungshemmenden Wirkung;
einen Sprüher (20), der ausgebildet ist, die Nutzsubstanz in einen Raum (S) zu sprühen, in dem sich eine Zielperson (T) befindet;
eine Steuereinheit (30), die ausgebildet ist, den Sprüher (20) zu steuern;
eine Feuchtigkeitssteuereinheit (14), die ausgebildet ist, eine Feuchtigkeit von Luft in dem Raum (S) zu steuern; und
einen Speicher (31), der ausgebildet ist, Daten zu speichern, die die Nutzsubstanz und eine mit der Nutzsubstanz assoziierte Soll-Feuchtigkeit einschließen, wobei
die durch den Sprüher (20) gesprühte Nutzsubstanz einen Partikeldurchmesser von 1 µm bis 10 µm einschließlich aufweist, und
die Steuereinheit (30) die mit der durch den Sprüher (20) gesprühten Nutzsubstanz assoziierte Soll-Feuchtigkeit, basierend auf den in dem Speicher (31) gespeicherten Daten, erfasst und die Feuchtigkeitssteuereinheit (14) so steuert, dass die Soll-Feuchtigkeit erfüllt wird.

2. Sprühvorrichtung nach Anspruch 1, wobei
die durch den Sprüher (20) gesprühte Nutzsubstanz einen Partikeldurchmesser von 5 µm bis 10 µm einschließlich aufweist.

3. Sprühvorrichtung nach Anspruch 1 oder 2, weiter umfassend:
eine Feuchtigkeitssteuereinheit (14), die ausgebildet ist, eine Feuchtigkeit von Luft in dem Raum (S) zu steuern, wobei
die Steuereinheit (30) die Feuchtigkeitssteuereinheit (14) so steuert, dass die Feuchtigkeit des Raums (S) während des Betriebs des Sprühers (20) 50 % oder mehr wird.

4. Sprühvorrichtung nach Anspruch 3, wobei
die Steuereinheit (30) die Feuchtigkeitssteuereinheit (14) so steuert, dass die Feuchtigkeit des Raums (S) während des Betriebs des Sprühers (20) 70 % oder mehr und 85 % oder weniger wird.

5. Sprühvorrichtung nach einem der Ansprüche 1 bis 4, weiter umfassend:
einen Positionssensor (16), der ausgebildet ist, eine Position einer Nase der Zielperson (T) zu detektieren; und
eine Richtungsänderungseinheit (13), die ausgebildet ist, eine Richtung zu ändern, in der die Nutzsubstanz durch den Sprüher (20) gesprüht wird, wobei
die Steuereinheit (30) die Richtungsänderungseinheit (13) so antreibt, dass die Nutzsubstanz in Übereinstimmung mit einem Ergebnis der Detektion der Position durch den Positionssensor (16) in Richtung der Nase der Zielperson (T) gesprüht wird.

6. Sprühvorrichtung nach einem der Ansprüche 1 bis 5, weiter umfassend:
einen Biosensor (15), der ausgebildet ist, biologische Informationen der Zielperson (T) zu detektieren, wobei
die Nutzsubstanz eine antioxidative Wirkung aufweist, und
die Steuereinheit (30) den Sprüher (20) veranlasst, die Nutzsubstanz zu sprühen, wenn basierend auf der durch den Biosensor (15) detektierten biologischen Informationen bestimmt wird, dass der Parasympathikus der Zielperson (T) dominant ist.

7. Sprühvorrichtung nach Anspruch 6, wobei
die Nutzsubstanz mindestens ein Naturprodukt einschließt, ausgewählt aus Traube, schwarzem Reis, Blaubeere, Sesam, Brokkoli, Knoblauch, Spinat, Tomate, Wassermelone, Zitrusfrucht, Rosmarin, Oregano, Thymian, Sesamsamen, Tabaskopfeffer, Pfeffer, Ingwer, Wegerichsamen, und Osbeckia chinensis.

8. Sprühvorrichtung nach Anspruch 6 oder 7, wobei
die Nutzsubstanz mindestens eine Substanz einschließt, ausgewählt aus Anthocyanin, Sesaminol, Sulforaphan, Allicin, Lutein, Lycopin, Limonen, Rosmanol, Rosmarindiphenol, Protocatechusäure, Kaffeesäure, Rosmarinsäure, Thymol, Carvacrol, Capsaicin, Hydrocapsaicin, Piperin, Zingeron, Gingerol, Ingwerextrakt, Geniposidsäure und Osbekinsäure.

9. Sprühvorrichtung nach einem der Ansprüche 6 bis 8, wobei
die Nutzsubstanz Sulforaphan einschließt, und
die Steuereinheit (30) den Sprüher (20) veranlasst, die Nutzsubstanz so zu sprühen, dass eine Gesamtsprühmenge des Sulforaphans an einem Tag von 10 µmol bis 1000 µmol einschließlich beträgt.

10. Sprühvorrichtung nach einem der Ansprüche 1 bis 5, weiter umfassend:
einen Biosensor (15), der ausgebildet ist, biologische Informationen der Zielperson (T) zu detektieren, wobei
die Nutzsubstanz eine entzündungshemmende Wirkung aufweist, und
die Steuereinheit (30) den Sprüher (20) veranlasst, die Nutzsubstanz zu sprühen, wenn basierend auf der durch den Biosensor (15) detektierten biologischen Informationen bestimmt wird, dass der Sympathikus der Zielperson (T) dominant ist.

11. Sprühvorrichtung nach Anspruch 10, wobei
die Nutzsubstanz mindestens ein Naturprodukt einschließt, ausgewählt aus Zitronengras, rotem Thymian, Patschuli, Krauseminze, Eukalyptus, Teebaum, echtem Lavendel, Bourbon-Geranie, Wacholder, Kamille, Echter Kamille, Weihrauch, Zitrone, Ingwer, schwarzem Pfeffer, Nelke, Zimt, Oregano, Winterbohnenkraut, Sternanis, Zitronenmelisse, Citronella, Melisse, Zitronengummi und Eisenkraut.

12. Sprühvorrichtung nach Anspruch 10 oder 11, wobei
die Nutzsubstanz mindestens eine Substanz einschließt, ausgewählt aus Citral, Thymol, Patchoulol, Carvon, 1,8-Cineol, Terpinen-4-ol, Linalool, Geraniol, P-Citronellol, α-Pinen, Chamazulen, Famesen, Bisabolen, Eugenol, Carvacrol, Anethol, Citronellal, und Neral.

13. Sprühvorrichtung nach einem der Ansprüche 10 bis 12, wobei
die Nutzsubstanz ein Terpinen-4-ol einschließt, und
die Steuereinheit (30) den Sprüher (20) veranlasst, die Nutzsubstanz so zu sprühen, dass eine Gesamtsprühmenge des Terpinen-4-ols an einem Tag von 10 µmol bis 1000 µmol einschließlich beträgt.

14. Luftbehandlungsvorrichtung, umfassend die Sprühvorrichtung (10) nach einem der Ansprüche 1 bis 13.

## Revendications

1. Dispositif de pulvérisation comprenant :
une substance utile présentant un effet antioxydant ou un effet anti-inflammatoire ;
un pulvérisateur (20) configuré pour pulvériser la substance utile dans un espace (S) où se trouve une personne cible (T) ;
un dispositif de commande (30) configuré pour commander le pulvérisateur (20) ;
une unité de régulation d'humidité (14) configurée pour réguler une humidité de l'air dans l'espace (S) ; et
une mémoire de stockage (31) configurée pour stocker des données incluant la substance utile et une humidité réglée associée à la substance utile, dans lequel
la substance utile pulvérisée par le pulvérisateur (20) présente un diamètre de particule de 1 µm à 10 µm inclus, et
le dispositif de commande (30) acquiert l'humidité réglée associée à la substance utile pulvérisée par le pulvérisateur (20) sur la base des données stockées dans la mémoire de stockage (31) et commande l'unité de régulation d'humidité (14) de manière à satisfaire l'humidité réglée.

2. Dispositif de pulvérisation selon la revendication 1, dans lequel
la substance utile pulvérisée par le pulvérisateur (20) présente un diamètre de particule de 5 µm à 10 µm inclus.

3. Dispositif de pulvérisation selon la revendication 1 ou la revendication 2, comprenant en outre :
une unité de régulation d'humidité (14) configurée pour réguler une humidité de l'air dans l'espace (S), dans lequel
le dispositif de commande (30) commande l'unité de régulation d'humidité (14) de sorte que l'humidité de l'espace (S) devienne de 50 % ou plus pendant le fonctionnement du pulvérisateur (20).

4. Dispositif de pulvérisation selon la revendication 3, dans lequel
le dispositif de commande (30) commande l'unité de régulation d'humidité (14) de sorte que l'humidité de l'espace (S) devienne de 70 % ou plus et de 85 % ou moins pendant le fonctionnement du pulvérisateur (20).

5. Dispositif de pulvérisation selon l'une quelconque des revendications 1 à 4, comprenant en outre :
un capteur de position (16) configuré pour détecter une position du nez de la personne cible (T) ; et
une unité de changement de direction (13) configurée pour changer une direction dans laquelle la substance utile est pulvérisée par le pulvérisateur (20), dans lequel
le dispositif de commande (30) entraîne l'unité de changement de direction (13) de sorte que la substance utile soit pulvérisée vers le nez de la personne cible (T) conformément à un résultat de la détection de la position par le capteur de position (16).

6. Dispositif de pulvérisation selon l'une quelconque des revendications 1 à 5, comprenant en outre :
un biocapteur (15) configuré pour détecter des informations biologiques de la personne cible (T), dans lequel
la substance utile présente un effet antioxydant, et
le dispositif de commande (30) amène le pulvérisateur (20) à pulvériser la substance utile s'il est déterminé, sur la base des informations biologiques détectées par le biocapteur (15), que le nerf parasympathique de la personne cible (T) est dominant.

7. Dispositif de pulvérisation selon la revendication 6, dans lequel
la substance utile inclut au moins un produit naturel sélectionné parmi raisin, riz noir, myrtille, sésame, brocoli, ail, épinard, tomate, pastèque, agrume, romarin, origan, thym, graine de sésame, piment tabasco, poivre, gingembre, graine de plantago, et osbeckia chinensis.

8. Dispositif de pulvérisation selon la revendication 6 ou la revendication 7, dans lequel
la substance utile inclut au moins une substance sélectionnée parmi anthocyanine, sésaminol, sulforaphane, allicine, lutéine, lycopène, limonène, rosmanol, diphénol de romarin, acide protocatéchuique, acide caféique, acide rosmarinique, thymol, carvacrol, capsaïcine, hydrocapsaïcine, pipérine, zingérone, gingérol, gingembre all, acide géniposidique, et acide osbekique.

9. Dispositif de pulvérisation selon l'une quelconque des revendications 6 à 8, dans lequel
la substance utile inclut du sulforaphane, et
le dispositif de commande (30) amène le pulvérisateur (20) à pulvériser la substance utile de sorte qu'une quantité totale pulvérisée du sulforaphane en une journée soit de 10 µmol à 1000 µmol inclus.

10. Dispositif de pulvérisation selon l'une quelconque des revendications 1 à 5, comprenant en outre :
un biocapteur (15) configuré pour détecter des informations biologiques de la personne cible (T), dans lequel
la substance utile présente un effet anti-inflammatoire, et
le dispositif de commande (30) amène le pulvérisateur (20) à pulvériser la substance utile s'il est déterminé, sur la base des informations biologiques détectées par le biocapteur (15), que le nerf sympathique de la personne cible (T) est dominant.

11. Dispositif de pulvérisation selon la revendication 10, dans lequel
la substance utile inclut au moins un produit naturel sélectionné parmi citronnelle, thym rouge, patchouli, menthe verte, eucalyptus, arbre à thé, lavande vraie, géranium bourbon, genévrier, camomille, camomille sauvage, encens, citron, gingembre, poivre noir, clou de girofle, cannelle, origan, sarriette des montagnes, anis étoilé, mélisse lemon balm, citronnelle, mélisse officinale, eucalyptus citronné, et verveine.

12. Dispositif de pulvérisation selon la revendication 10 ou la revendication 11, dans lequel
la substance utile inclut au moins une substance sélectionnée parmi citral, thymol, patchoulol, carvone, 1,8-cinéole, terpinène-4-ol, linalol, géraniol, β-citronellol, α-pinène, chamazulène, farnésène, bisabolène, eugénol, carvacrol, anéthole, citronellal, et néral.

13. Dispositif de pulvérisation selon l'une quelconque des revendications 10 à 12, dans lequel
la substance utile inclut un terpinène-4-ol, et
le dispositif de commande (30) amène le pulvérisateur (20) à pulvériser la substance utile de sorte qu'une quantité totale pulvérisée du terpinène-4-ol en une journée soit de 10 µmol à 1000 µmol inclus.

14. Dispositif de traitement d'air comprenant le dispositif de pulvérisation (10) selon l'une quelconque des revendications 1 à 13.
